# EUROPEAN PATENT SPECIFICATION

(11) **EP 1 890 998 B1**
(45) Date of publication and mention of the grant of the patent: **26.02.2014**
(21) Application number: 06771056.6
(22) Date of filing: 23.05.2006
(51) Int. Cl.: C07C 263/10, C07C 263/20, C07C 265/14, C08G 18/76

(54) **PROCESS FOR THE PREPARATION OF POLYISOCYANATES OF THE DIPHENYLMETHANE SERIES**
VERFAHREN FÜR DIE ZUBEREITUNG VON POLYISOCYANATEN DER DIPHENYLMETHAN-SERIE
PROCEDE DE PREPARATION DE POLYISOCYANATES DE LA SERIE DIPHENYLMETHANE

(30) Priority: 30.05.2005 EP 05104608
(43) Date of publication of application: 27.02.2008
(73) Proprietor: HUNTSMAN INTERNATIONAL LLC, Salt Lake City, Utah 84108 (US)
(72) Inventor: SMITH, Richard C., Glasinfryn, Bangor Gwynedd LL57 4UP (GB); CARR, Robert H., B-3060 Bertem (BE)
(74) Representative: Swinnen, Anne-Marie
(86) International application number: PCT/US2006/020065
(87) International publication number: WO 2006/130405

(56) References cited:
- WO-A-01/00569
- WO-A-2004/056756
- DE-A1- 4 232 769
- DE-A1- 10 245 584
- US-B1- 6 576 788

## Description

The present invention relates to a process for preparing mixtures of diphenylmethane diisocyanates and polyphenylpolymethylene polyisocyanates, known as PMDI, having a higher HunterLab color (L) number by reaction of the corresponding mixtures of diphenylmethanediamines and polyphenylpolymethylenepolyamines, known as PMDA, with phosgene in the presence of at least one inert organic solvent.

PMDI is an industrially important isocyanate for producing rigid polyurethane foams which are preferably used as insulation material in the building industry, as insulating foam in the refrigeration appliance industry and as sandwich panel construction material. Usually, part of the diphenylmethane 4,4'-diisocyanate, known as MMDI, present in the PMDI, is recovered by means of a suitable technological operation such as distillation or crystallization. MMDI is in turn an important constituent of polyurethane formulations for compact, microcellular and cellular polyurethanes such as adhesives, coatings, fibers, elastomers and integral foams. Likewise, various mixtures of the diisocyanate isomers in varying proportions (so-called "Mixed Isomer" products) can be prepared. Accordingly, the term "PMDI" as used herein also encompasses PMDI mixtures in which monomeric MDI, for example 4,4'-, 2,2'- and/or 2,4'-MDI, is present.

Historically, PMDI was made by initial reaction of the corresponding PMDA in an inert organic solvent with either hydrogen chloride or carbon dioxide to make a suspension of the amine salts, followed by reaction with phosgene. However, these methods are uneconomic because of the very long reaction time resulting from complete conversion of the PMDA to salt forms. PMDI is, as is known, now widely prepared industrially by direct phosgenation of the PMDA in the presence of an inert organic solvent. PMDA is in turn obtained by means of an acid catalysed aniline-formaldehyde condensation which can be carried out industrially either continuously or batchwise. The proportions of diphenylmethanediamines and the homologous polyphenylpolymethylenepolyamines and their positional isomers in the PMDA are controlled by selection of the ratios of aniline, formaldehyde and acid catalyst and also by means of a suitable temperature and residence time profile. High contents of 4,4'-diphenylmethanediamine together with a simultaneously low proportion of the 2,4' isomer of diphenylinethanediamine are obtained on an industrial scale by the use of strong mineral acids such as hydrochloric acid as catalyst in the aniline-formaldehyde condensation. Use of a wide range of solid acid catalysts is also known.

The final color of MDI products is the combined result of a number of different effects based on different chemistries. For example, the presence of colour in organic products caused by traces of halogenated impurities, especially brominated or iodinated impurities, is known and minimising such impurities in the manufacture of MDI leads to products with improved colour (US 6900348). Reaction of oxygen with the polyamine (PMDA) precursor to MDI can ultimately lead to the formation of quinone-imine-type impurities which are similar to highly colored arylmethine dyes (Color Chemistry - Synthesis, Properties and Applications of Organic Dyes and Pigments, H. Zollinger, Wiley-VCH ISBN 3-906390-23-3) which can also give chromophores in the MDI after the phosgenation and work-up processes. Other impurities from process steps preceding phosgenation may also contribute to the final color of MDI. For example, all the acid catalysed aniline-formaldehyde condensation processes described in the specialist and patent literature have in common the formation of undesired by-products, for example the formation of N-methylated and N-formylated compounds and also the formation of dihydroquinazolines. In addition, industrial PMDAs can contain residual amounts of unrearranged aminobenzylanilines which can in turn be a further starting point for further reactions. Another disadvantage is that the acid aniline-formaldehyde condensation forms chromophores which discolor the PMDA. These discolorations are reduced only insufficiently, if at all, in the subsequent neutralization of the acid condensation catalyst and the removal of the aniline used in excess in the condensation; the same applies to the subsequent process steps of the PMDI preparation.

In the conversion of the PMDA to PMDI, the PMDA is reacted with phosgene, typically in the presence of an inert organic solvent. This now widely operated process can be described according to various stages although, in the prior art, different authors have defined the stages in a number of different ways. The stages may be described according to the chemical changes occurring in the reacting mixture or according to the manufacturing process conditions or equipment being used or as a combination of these.

After suitable preparation of the various reaction components, the chemical process of converting PMDA to PMDI begins with the initial reaction of amine and phosgene, producing carbamoyl chlorides and HCl. Well known side reactions are the formation of a range of urea-group containing compounds and insoluble and heat-stable amine hydrochlorides, whose exact composition is related to the particular amine feed composition and the particular process configuration (pressure, temperature, mixing regime, etc) used at this stage. The resulting mixture may be reacted further in the same vessel or may be discharged from one reactor to a subsequent reactor for the further stage(s) of manufacture, where the thermally-sensitive carbamoyl chlorides can be decomposed to isocyanate and HCl by increasing the temperature of the mixture and solid amine hydrochlorides are converted to isocyanate by further reaction with phosgene. Reactor in this context can be any type of vessel (stirred tank reactors, plug-flow reactors such as tower reactors or, indeed, any device which can be used for the contacting of reactants, at this stage, the still-to-react components being carbamoyl chlorides, amine hydrochlorides and phosgene). Thus, there exist many devices and combinations of devices for carrying out the staged conversion of amine feed to the corresponding isocyanate product, by reaction with phosgene and co-formation of HCl, optionally in a solvent, and subsequent removal of excess phosgene, HCl and solvent, thermal breakdown of chlorinated impurities and removal of minor volatile impurities. For example, WO 2004/056756 and DE 10245584 describe specific process configurations which address specific issues in the complex production process, the object being to improve process operations and efficiency, rather than to improve product quality.

The reactions taking place in each stage of the phosgenation run are the following:

### First stage:

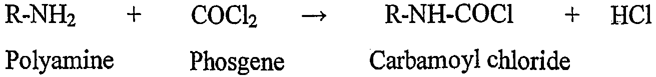

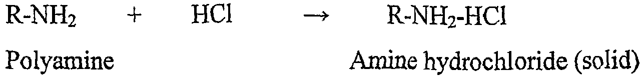

### Subsequent stage:

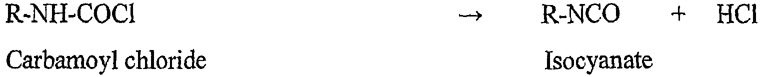

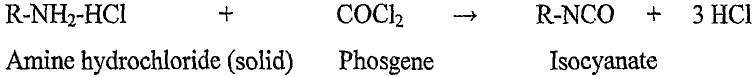

The undesired by-products and chromophores in the PMDA can react with phosgene to form further compounds such as secondary carbamoyl chlorides and products of chlorination of the aromatic ring and/or at the methylene bridge. In addition, the phosgenation step forms further chlorine-containing by-products such as allophanoyl chlorides and isonitrile dichlorides. The chlorine-containing compounds and chromophores are incorporated both into the low molecular weight fraction whose central constituent is the diphenylmethane diisocyanate and also into the oligomeric fractions of polyphenylpolymethylene polyisocyanate.

The technological operations which follow the phosgenation, namely removal of the phosgene used in excess, the removal of the inert solvent, the thermal treatment, the so-called dechlorination and the removal of part of the MMDI present in the crude PMDI by distillation and/or crystallization, do not lastingly reduce the discoloration of the crude PMDI and the discoloration of the crude PMDI increases with continuing, especially thermal, stressing of the product.

Discolored PMDI is undesirable in further processing to form polyisocyanate-polyalcohol polyaddition plastics. In particular, undesirable discolorations of the PMDI can show up in the plastics prepared therefrom. Although the color of the polyisocyanate-polyalcohol polyaddition plastics does not have an adverse effect on their mechanical properties, light-colored products are preferred because of their good versatility in the production process of the processor, e.g. the ability of light to pass through thin covering layers and the ability to produce a variety of colors.

There have therefore been many attempts to reduce the discoloration of PMDI in mixtures with MMDI.

To lighten PMDI color, special additional treatments of the PMDA have been proposed such as mild partial catalytic reduction (as in EP 546 400 and US 5 889 070), re-acidification (e.g. US 5 386 059), extra base treatment (see DE 1 0211 021). Such additional treatments add significantly to the complexity of the PMDA preparation process and are unsatisfactory on economic grounds.

To lighten PMDI color, the addition of numerous compounds before, during or after the phosgenation reaction has also been proposed. Many examples of such added compounds can be characterised by the presence of functional groups (especially -OH, -NH, -NH₂) which react readily with phosgene and include water (US 4 465 639), low molecular weight monohydric or polyhydric alcohols (EP 445 602), polyether polyols or alkane polyols (US 4 507 464), water and alcohols (US 6 229 043), phenol derivatives (DE 4 300 774), amines and/or ureas (DE 4 232 769), polyoxyalkylene polyalcohols (DE 4 021 712), hydrazine or derivatives (US 5 942 151). Other chemicals used include acid chlorides and chloroformates (DE 4 118 914), carboxylic acids (EP 538 500), dialkyl or trialkyl phosphites (DE 4 006 978), organic phosphorous acid (JP 3 292 857), acid chlorides/antioxidant (DE 4 318 018), special reducing agents (US 5 312 971). Improving the colour of MDI products by using amine hydrochlorides has not been disclosed previously. All processes which propose the addition of compounds to raw materials or products of a preparation stage for PMDI have the disadvantage of the addition of an additional agent with the inherent danger of its corrosive action on the equipment components and the formation of by-products from precisely these added agents, which by-products can in turn have an adverse effect on the product or the equipment. Such additional treatments also add significantly to the complexity of the PMDI preparation process and are unsatisfactory on economic grounds.

To lighten PMDI color, special additional treatments of the PMDI have also been proposed: hydrogenation (EP 816 333, US 5 583 251 and US 6 140 382), irradition with light (US 5 994 579), heat treatment with hydrogen chloride (US 5 364 958). Such additional treatments add significantly to the complexity of the PMDI preparation process and are unsatisfactory on economic grounds.

US Patent No. 4 876 380 proposes lightening the color by extraction of a chromophorerich PMDI fraction from the PMDI by means of pentane/hexane. Disadvantages of this process are the carrying-out of a complicated technological operation with additional steps for working up the extractant and the unavoidable formation of a reduced-quality PMDI fraction for which applications that use up equivalent amounts have to be found.

US Patent No. 6 576 788 proposes production of PMDI in a process where the mass ratios of phosgene to hydrogen chloride in the residence time apparatus of the second stage of the phosgenation are at the same time 10-30:1 in the liquid phase and 1-10:1 in the gas phase. Disadvantages of such a process are in the complexity of simultaneously measuring and controlling the different phase compositions to achieve the lightening.

Thus, there continues to be a need for a cost-effective method of improving the color of PMDI and PMDI-derived polyurethane materials without the drawbacks mentioned above.

It is an object of the present invention to lighten the color of the PMDI in admixture with MMDI while avoiding the above mentioned disadvantages. In particular, the addition of additional reagents and/or the use of additional apparatus should not be necessary.

We have found that this object is achieved by staged reaction of the corresponding mixtures comprising diphenylmethanediamines and polyphenylpolymethylenepolyamines with phosgene in the presence of at least one solvent as described in claims 1-6, where the corresponding carbamoyl chlorides and the amine hydrochlorides formed in the first stage of the phosgenation run through a subsequent stage of the phosgenation apparatus in which the carbamoyl chlorides are dissociated into the corresponding isocyanates and hydrogen chloride and some amine hydrochlorides remain unreacted.

The present invention accordingly provides a process for preparing mixtures comprising diphenylmethane diisocyanates and polyphenylpolymethylene polyisocyanates having a higher HunterLab color (L) number by staged reaction of the corresponding mixtures comprising diphenylmethanediamines and polyphenylpolymethylenepolyamines with phosgene in the presence of at least one solvent, where the corresponding carbamoyl chlorides and the amine hydrochlorides formed in the first stage of the phosgenation run through a subsequent stage of the phosgenation apparatus in which some amine hydrochlorides remain unreacted and the carbamoyl chlorides are dissociated into the corresponding isocyanates and hydrogen chloride.

PMDI color has historically been quoted according to several different color scales. Here we use the HunterLab system, where L is the Lightness. Further information on this and other color scales is widely available in the literature, for example, in "The Measurement of Appearance", R.S. Hunter & R. W. Harold, John Wiley & Sons (ISBN 0-471-83006-2).

The phosgenation of primary amines in a mixing reactor as first stage of the phosgenation has been described a number of times. Thus, for example, US 3 544 611 and EP 150 435 report the phosgenation in a pressure mixing circuit. Furthermore, EP 291 819 discloses carrying out this reaction in a reaction pump. Many different designs of static mixers have been described, for example: annular slot nozzle (FR 2 325 637, DE 1 792 660), ring-eye nozzle (DE 3 744 001), flat jet nozzle (EP 65 727), fan jet nozzle (DE 2 950 216), angle-jet chamber nozzle (DD 300 168), three-fluid nozzle (DD 132 340), coaxial jet mixer nozzle with protruding centerbody (US 2004008572). The temperature in the first stage of the phosgenation is usually from 40 to 150°C, preferably from 60 to 130°C, particularly preferably from 90 to 120°C. By allowing the exothermic reactions taking place to increase the temperature of the mixture to above approximately 80°C, solidification of carbamoyl chlorides can be prevented (US 2006/0041166). Careful design of the mixing device minimises urea by-product formation by minimising contacting of incoming amine with reaction products, such that formation of insoluble "polyureas" is avoided. Formation of some urea functional groups is not problematic since these will be simultaneously present in compounds also containing polyisocyanates and, thus, such "mixed functionality" compounds will be soluble in the mixture of normal polyisocyanates.

In a subsequent stage the corresponding carbamoyl chlorides and amine hydrochlorides formed in the first stage of the phosgenation can be run through many types of residence time apparatus in which the amine hydrochlorides are phosgenated to form the corresponding carbamoyl chlorides and the carbamoyl chlorides are dissociated into the corresponding isocyanates and hydrogen chloride. For example, the mixture from a previous stage of the phosgenation can be fed to a series of stirred tank reactors, tubular or column reactors or thin film devices (such as in WO 2004031132) or combinations of different types of reactors. Batch, continuous, semi-continuous processes and combinations of these, operating at atmospheric pressure or above, are all known in the art.

The PMDI mixtures prepared by the process of the present invention usually have a diphenylmethane diisocyanate isomer content of from 30 to 90% by weight, preferably from 30 to 70% by weight, an NCO content of from 29 to 33% by weight, preferably from 30 to 32% by weight, based on the weight of crude MDI, and a viscosity, determined at 25°C in accordance with DIN 51550, of not more than 2500 mPa.s, preferably from 40 to 2000 mPa.s.

Crude PMDIs having such isomer and homologue compositions can be prepared by phosgenation of crude PMDAs having corresponding product compositions in the presence of at least one solvent.

Suitable crude PMDAs are advantageously obtained by condensation of aniline and formaldehyde in a molar ratio of 6-1.6:1, preferably 4-1.9:1, and a molar ratio of aniline to acid catalysts of 1:0.98-0.01, preferably 1:0.8-0.1.

The formaldehyde can be used in any physical form (solid, liquid or gas) and is preferably used in the form of an aqueous solution, e.g. as a commercial 30-55 % strength by mass solution.

Acid catalysts which have been found to be useful are proton donors such as acid ion exchange resins or strong organic and preferably inorganic acids. For the purposes of the present invention, strong acids are those having a pKa of less than 1.5; in the case of polybasic acids, this value is that for the first hydrogen dissociation. Examples which may be mentioned are hydrochloric acid, sulfuric acid, phosphoric acid, fluorosulfonic acid and oxalic acid. Hydrogen chloride in gaseous form can also be used. Preference is given to using aqueous hydrochloric acid in concentrations of from about 25 to 33% by mass.

Suitable processes for preparing crude PMDA are described, for example, in CA 700 026, DE 22 27 110 (US 4 025 557), DE 22 38 920 (US 3 996 283), DE 24 26 116 (GB 1 450 632), DE 12 42 623 (US 3 478 099), GB 1 064 559 and DE 32 25 125.

The other starting component for preparing crude PMDI is phosgene. The phosgene can be used as liquid or gas, diluted in solvents or with other gases which are inert under the reaction conditions, e.g. monochlorobenzene, ortho dichlorobenzene, nitrogen, carbon monoxide, etc. The molar ratio of crude PMDA to phosgene is advantageously selected such that from 1 to 10 mol, preferably from 1.2 to 4 mol, of phosgene are present in the reaction mixture per mole of NH₂ groups. The phosgene can all be fed into the first stage of the phosgenation or part of it can also be added to the residence time apparatus of the subsequent stage of the phosgenation.

Suitable solvents are compounds in which the crude PMDA and the phosgene are at least partially soluble. Solvents which have been found to be useful are chlorinated, aromatic hydrocarbons, for example monochlorobenzene, dichlorobenzenes such as o-dichlorobenzene and p-dichlorobenzene, trichlorobenzenes, the corresponding toluenes and xylenes, chloroethylbenzene, monochlorobiphenyl, alpha- or beta-naphthyl chloride and dialkyl phthalates such as diethyl isophthalate. Isocyanate compounds or mixtures other than MDI's or, preferably, crude or purified PMDI or other MDI material can also be used to replace some or all of the non-isocyanate solvent after the crude PMDA has been initially reacted with the phosgene. Excess phosgene can also be used to take the role of the solvent. Particular preference is given to using monochlorobenzene (MCB), dichlorobenzenes or mixtures of these chlorobenzenes as inert organic solvents. The solvents can be used individually or as mixtures. It is advantageous to use a solvent which has a boiling point lower than that of the MDI isomers so that the solvent can easily be separated from the crude PMDI by distillation. The amount of solvent is advantageously selected such that the reaction mixture has an isocyanate content of from 2 to 40% by mass, preferably from 5 to 20% by mass, based on the total weight of the reaction mixture.

The crude PMDA can be employed as such or as a solution in organic solvents. However, particular preference is given to using crude PMDA solutions having an amine content of from 2 to 45% by mass, preferably from 25 to 44% by mass, based on the total weight of the amine solution.

Dependent upon the exact design of the phosgenation reaction section and the conditions of temperature and pressure selected, varying proportions of phosgene, hydrogen chloride, solvent and other components of the complex reaction mixture will be partitioned between vapor, solution and solids phases. The vapor phase may be largely or partially separated from or may be kept in direct contact with the solution and solids during different stages of the phosgenation.

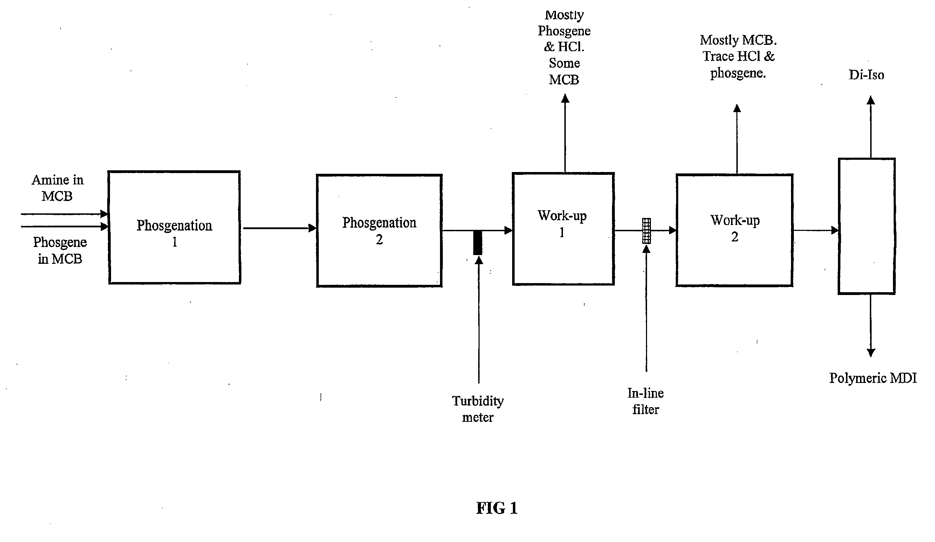

Subsequent to the phosgenation stages, the reaction mixture is worked-up such that remaining excess phosgene and hydrogen chloride and the solvent are preferably separated from the reaction product (Figure 1). The work-up procedure also includes a thermal treatment step (the so-called "dechlorination") which is likewise well known in the art. The crude PMDI may then be further treated to produce diisocyanate and polymeric MDI products.

To prepare a PMDI having a higher HunterLab color (L) number, it has surprisingly been discovered to be particularly advantageous to operate the process such that a controlled concentration of amine hydrochloride solids remains unreacted at the end of the phosgenation reactors so that these solids are present whilst residual phosgene is being removed from the reaction mixture, in contrast to previous prior art where work-up takes place after the chemical reactions of phosgenation are complete or where the concentration of amine hydrochlorides is not specifically controlled or where additional chemical treatments, disadvantageously requiring additional process equipment, are required (for example CA 2 180 285, DE 4 318 018, DE 4 232 769, CA 2 046 365, EP 0445 602).

The residual content of amine hydrochloride solids at this time is between 10 and 5000 ppm, preferably 1500 to 2500 ppm. The higher the solids content, the better the color. A too high solids content could lead to filter blocking downstream. Solid carbamoyl chlorides and solid ureas are not present. Residual phosgene in the liquid phase at this point is between 0.5 and 5 wt%, preferably between 1 and 2 wt%.

The amine hydrochloride solids content is monitored, preferably continuously with on-line measurement devices (light scattering detectors, laser based devices, ultrasound devices and the like) which have been calibrated by methods known to those skilled in the art.

The mixture of phosgene, hydrogen chloride, solvent, amine hydrochlorides, isocyanates and various impurities is subsequently subjected to rapid heating from about 90-100°C to about 130-140°C within a time period of less than about 20 seconds, either by passing the mixture through a heat exchanging device or by recycling hot isocyanate from elsewhere in the work-up section; the latter leading to nearly instantaneous heating.
The presence of solid amine hydrochlorides at this stage is surprisingly beneficial for the subsequent color of the PMDI.

Downstream of this first work-up section is an in-line filter which protects the remainder of the production equipment from solids which might, for example, cause blockage. The solids level here is then reduced to almost zero.

This controlled level of amine hydrochlorides entering the first work-up section is largely consumed by reaction with phosgene, simultaneously with phosgene removal down to low levels. Final traces of residual HCl, phosgene and MCB are removed in a further work-up section. For practical reasons, in case of loss of control of the process, the second work-up section and subsequent operations are protected from break-through of excess solids by any suitable means, for example, in-line filters.

The final level of phosgene after the phosgene removal is <10 ppm of phosgene. These work-up steps are carried out by generally known methods. The diisocyanate isomer products can be separated from the PMDI mixture by known methods such as distillation or crystallization, with or without additional work-up treatment.

The product may then be stabilized using an antioxidant based on sterically hindered phenols and/or at least one aryl phosphite. The stabilizers are advantageously used in an amount of up to max. 1% by mass, preferably from 0.001 to 0.2% by mass. Examples of suitable antioxidants based on sterically hindered phenols are: styrenized phenols, i.e. phenols which have a 1-phenylethyl group bound in the 2 or 4 position or in the 2 and 4 and/or 6 positions, bis(2-hydroxy-5-methyl-3-tert-butylphenyl)methane, 2,2-bis(4-hydroxyphenyl)propane, 4,4,'-dihydroxybiphenyl, 3,3'-dialkyl- or 3,3',5,5'-tetraalkyl-4,4'-dihydroxybiphenyl, bis(4-hydroxy-2-methyl-5-tert-butylphenyl)sulfide, hydroquinone, 4-methoxy-, 4-tert-butoxy- or 4-benzyloxy-phenol, mixtures of 4-methoxy-2- or -3-tertbutylphenol, 2,5-dihydroxy-1-tert-butylbenzene, 2,5-dihydroxy-1,4-di-tert-butylbenzene, 4-methoxy-2,6-di-tert-butylphenol and preferably 2,6-di-tert-butyl-p-cresol.

Aryl phosphites which have been found to be useful are tri(alkylphenyl)phosphites having from 1 to 10 carbon atoms in the alkyl radical, e.g. tri(methylphenyl)phosphite, tri(ethylphenyl)phosphite, tri(n-propylphenyl)phosphite, tri(isopropylphenyl)phosphite, tri(n-butylphenyl)phosphite, tri(sec-butylphenyl)phosphite, tri(tert-butylphenyl)phosphite, tri(pentylphenyl)phosphite, tri(hexylphenyl)phosphite, tri(2-ethylhexylphenyl)phosphite, tri(octylphenyl)phosphite, tri(2-ethyloctylphenyl)phosphite, tri(decylphenyl)phosphite and preferably tri(nonylphenyl)phosphite, and in particular triphenyl phosphite.

Using the process of the present invention leads to PMDI having lighter color, typically having a HunterLab color (L) number of greater than 10, preferably greater than 25, more preferably greater than 40 and, typically, in the range 20 to 50. Color can be determined using laboratory instruments on samples of final product or crude PMDI or, preferably, by means of in-line process analysers which give additional benefits such as minimising sampling of process streams and of providing rapid data to enhance process control, either manually or automatically.

The invention is illustrated by the following example:

### EXAMPLE

A PMDA containing about 62 percent by weight diamine isomers (4,4', 2,4', 2,2'isomer ratio of 90.7/9.0/0.3) and decreasing amounts of the higher polyamine homologues was prepared by hydrochloric acid catalysed condensation of aniline and formaldehyde (in aqueous solution as formalin). Low levels of the normal impurities (N-methylated, N-formylated and dihydroquinazoline-containing compounds) were also present. After removal of excess aniline and water, the PMDA was dissolved in monochlorobenzene (MCB) to give a solution of about 27 per cent by weight polyamine which was continuously introduced together with an approximately 60 per cent by weight solution of phosgene in MCB into the first stage of a phosgenation apparatus, which is a static mixing nozzle device. The exothermic reactions taking place are allowed to increase the temperature of the mixture to approximately 80-95 °C, such that solidification of carbamoyl chlorides is prevented.

The reaction mixture from this first stage entered the subsequent stage of phosgenation which is a series of continuously stirred tank reactors to convert residual carbamoyl chlorides into the corresponding isocyanates and almost completely react away solid amine hydrochlorides.

The solids content in the mixture leaving the phosgenation was measured by an in-line turbidity meter (Optek Turbidity meter model 516/TF16), which had been calibrated using formazin suspensions of known concentrations (see USEPA Method 180.1).
The reaction mixture then entered the first work-up section and was heated from about 90-100°C to about 130-140°C within a time period of about 15 seconds by passing the mixture through a heat exchanging device. In this temperature range, phosgene and HCl are almost totally removed to the vapor handling system.
After passing through the in-line filter into the second work-up section, the crude PMDI was finally freed of all phosgene, HCl and MCB and thermally treated (the so-called "dechlorination") in accordance with the prior art. The crude PMDI was further treated by partial fractional distillation to produce diisocyanate and polymeric MDI product. The diisocyanate fraction was further treated by fractional crystallisation to produce MMDI and mixed isomer fractions.

The color of the final polymeric MDI product was measured using a HunterLab instrument. The relationship between the parts per million level of solid amine hydrochloride determined by the calibrated turbidity meter and the color of the final polymeric MDI product is demonstrated by the following results :-

| Amine hydrochloride solids (ppm) | Polymeric MDI color (HunterLab L) |
|---|---|
| | |
| 2186 | 22.3 |
| 2065 | 21.3 |
| 1953 | 19.6 |
| 1823 | 15.3 |

The solid levels quoted in the table above are defined in terms of the turbidity meter calibration units i.e. the parts per million values of amine hydrochloride are quoted relative to the parts per million values of the formazin calibration procedure.

## Claims

1. A process for preparing mixtures comprising diphenylmethane diisocyanates and polyphenylpolymethylene polyisocyanates comprising the step of reacting the corresponding mixtures of diphenybnethanediamines and polyphenylpolymethylenepolyamines with phosgene in the presence of at least one solvent in stages whereby in a first stage the corresponding carbamoyl chlorides and amine hydrochlorides are formed and whereby in a subsequent stage residual carbamoyl chlorides are dissociated into the corresponding polyisocyanates and hydrogen chloride and amine hydrochlorides are phosgenated to form ultimately the corresponding polyisocyanates **characterised in that** a controlled amount of amine hydrochloride solids remain unreacted at the point where residual excess phosgene is removed from the reaction mixture, wherein the residual content of amine hydrochloride solids at the point where residual excess phosgene is removed from the reaction mixture is between 10 and 5000 ppm, and wherein said solid carbamoyl chlorides and/or solid ureas are not present at the point where residual excess phosgene is removed from the reaction mixture.

2. Process according to claim 1 wherein the residual content of amine hydrochloride solids at the point where residual excess phosgene is removed from the reaction mixture is between 1500 to 2500 ppm.

3. Process according to any one of the preceding claims wherein the amine hydrochloride solids content is measured by on-line analysis using light scattering or laser-based devices or techniques based on sonics or ultrasound.

4. Process according to any one of the preceding claims wherein the polyisocyanate mixture has a diphenylmethane diisocyanate isomer content of from 30 to 90% by weight, an isocyanate content of from 29 to 33% by weight and a viscosity of not more than 2500 mPa.s at 25 °C.

5. Process according to any one of the preceding claims wherein subsequent to the phosgenation reaction the excess phosgene, the hydrogen chloride and the solvent are separated from the reaction product.

6. Process according to claim 5 wherein the reaction mixture is subsequently further treated to produce diphenylmethane diisocyanates and polyphenylpolymethylene polyisocyanates.

## Patentansprüche

1. Verfahren zum Herstellen von Gemischen, die Diphenylmethandiisocyanate und Polyphenylpolymethylenpolyisocyanate aufweisen, das den Schritt des stufenweisen Umsetzens der entsprechenden Gemische von Diphenylmethandiaminen und Polyphenylpolymethylenpolyaminen mit Phosgen in Gegenwart von zumindest einem Lösungsmittel aufweist, wobei in einer ersten Stufe die entsprechenden Carbamoylchloride und Aminhydrochloride gebildet werden und wobei in einer nachfolgenden Stufe restliche Carbamoylchloride in die entsprechenden Polyisocyanate und Chlorwasserstoff dissoziieren und Aminhydrochloride phosgeniert werden, um schließlich die entsprechenden Polyisocyanate zu erzeugen,
**dadurch gekennzeichnet, dass** eine gesteuerte Menge fester Aminhydrochloride zu dem Zeitpunkt unreagiert verbleiben, zu dem das restliche überschüssige Phosgen aus dem Reaktionsgemisch entfernt wird, wobei der Restgehalt an festen Aminhydrochloriden zu dem Zeitpunkt, zu dem das restliche überschüssige Phosgen aus dem Reaktionsgemisch entfernt wird, 10 bis 5000 ppm beträgt und wobei die festen Carbamoylchloride und/oder festen Harnstoffe zu dem Zeitpunkt nicht vorliegen, zu dem das restliche überschüssige Phosgen aus dem Reaktionsgemisch entfernt wird.

2. Verfahren nach Anspruch 1,
wobei der Restgehalt an festen Aminhydrochloriden zu dem Zeitpunkt, zu dem das restliche überschüssige Phosgen aus dem Reaktionsgemisch entfernt wird, 1500 bis 2500 ppm beträgt.

3. Verfahren nach einem der vorstehenden Ansprüche,
wobei der Gehalt an festen Aminhydrochloriden durch direktgekoppelte Analyse unter Anwendung von auf Lichtstreuung oder Laser basierenden Vorrichtungen oder auf Schall oder Ultraschall basierenden Verfahren gemessen wird.

4. Verfahren nach einem der vorstehenden Ansprüche,
wobei das Polyisocyanatgemisch einen Gehalt an Diphenylmethandiisocyanat-Isomeren von 30 bis 90 Gew.-% und einen Isocyanatgehalt von 29 bis 33 Gew.-% und eine Viskosität von nicht mehr als 2500 mPa·s bei 25 °C aufweist.

5. Verfahren nach einem der vorstehenden Ansprüche,
wobei das überschüssige Phosgen, der Chlorwasserstoff und das Lösungsmittel nach der Phosgenierungsreaktion vom Reaktionsprodukt abgetrennt werden.

6. Verfahren nach Anspruch 5,
wobei das Reaktionsgemisch anschließend weiterbehandelt wird, um Diphenylmethandiisocyanate und Polyphenylpolymethylenpolyisocyanate zu erzeugen.

## Revendications

1. Procédé pour préparer des mélanges comprenant des diphénylméthane-diisocyanates et polyphénylpolyméthylène-polyisocyanates, comprenant l'étape de réaction des mélanges correspondants de phényléthanediamines et de polyphényl-polyméthylènepolyamines avec le phosgène en présence d'au moins un solvant par étapes, dans lequel, dans une première étape, les chlorures de carbamoyle et chlorhydrates d'amines correspondants sont formés et, ainsi, dans une étape ultérieure, les chlorures de carbamoyle résiduels sont dissociés en les polyisocyanates correspondants et chlorure d'hydrogène et les chlorhydrates d'amines sont phosgénés pour former finalement les polyisocyanates correspondants, **caractérisé en ce qu'**une quantité ajustée de chlorhydrates d'amines solides restent sans réaction au point où le phosgène en excès résiduel est éliminé du mélange réactionnel, la teneur résiduelle en chlorhydrates d'aminés solides au point où le phosgène en excès résiduel est éliminé du mélange réactionnel étant comprise entre 10 et 5000 ppm, et lesdits chlorures de carbamoyle solides et/ou urées solides ne sont pas présents au point où le phosgène résiduel est éliminé du mélange réactionnel.

2. Procédé suivant la revendication 1, dans lequel la teneur résiduelle en chlorhydrates d'amines solides au point où le phosgène en excès résiduel est éliminé du mélange réactionnel est comprise entre 1500 et 2500 ppm.

3. Procédé suivant l'une quelconque des revendications précédentes, dans lequel la teneur en chlorhydrates d'amines solides est mesurée par une analyse directe utilisant la diffusion de lumière ou des dispositifs à base de laser ou bien des techniques à base de sons ou d'ultrasons.

4. Procédé suivant l'une quelconque des revendications précédentes, dans lequel le mélange de polyisocyanates a une teneur en isomères de diphénylméthane-diisocyanate de 30 à 90 % en poids, une teneur en isocyanates de 29 à 33 % en poids et une viscosité non supérieure à 2500 mPa.s à 25°C.

5. Procédé suivant l'une quelconque des revendications précédentes, dans lequel, après la réaction de phosgénation, le phosgène en excès, le chlorure d'hydrogène et le solvant sont séparés du produit de réaction.

6. Procédé suivant la revendication 5, dans lequel le mélange réactionnel est ensuite soumis à un traitement supplémentaire pour produire des diphenylméthane-diisocyanates et polyphénylpolyméthylène-polyisocyanates.
